# EUROPEAN PATENT SPECIFICATION

(11) **EP 4 539 746 B1**
(45) Date of publication and mention of the grant of the patent: **31.12.2025**
(21) Application number: 23732442.1
(22) Date of filing: 08.06.2023
(51) Int. Cl.: A61B 8/00, A61B 8/08, G16H 30/20

(54) **METHODS AND SYSTEMS FOR AUTO-QUANTIFICATION OF ULTRASOUND IMAGES DURING LIVE SCAN**
VERFAHREN UND SYSTEME ZUR AUTOQUANTIFIZIERUNG VON ULTRASCHALLBILDERN WÄHREND EINER LIVE-ABTASTUNG
PROCÉDÉS ET SYSTÈMES D'AUTO-QUANTIFICATION D'IMAGES ULTRASONORES PENDANT UN BALAYAGE EN DIRECT

(30) Priority: 14.06.2022 US 202263351979 P; 10.10.2022 US 202263414720 P
(43) Date of publication of application: 23.04.2025
(73) Proprietor: Koninklijke Philips N.V., 5656 AG Eindhoven (NL)
(72) Inventor: BHARAT, Shyam, 5656 AG Eindhoven (NL); ERKAMP, Ramon Quido, 5656 AG Eindhoven (NL); BINGLEY, Peter, 5656 AG Eindhoven (NL)
(74) Representative: Philips Intellectual Property & Standards
(86) International application number: PCT/EP2023/065317
(87) International publication number: WO 2023/242038

(56) References cited:
- US-A1- 2019 130 554
- US-A1- 2021 330 285

## Description

### Field of the Disclosure

The present disclosure is directed generally to methods and systems for automated ultrasound quantification during an ultrasound scan.

### Background

Automation of ultrasound quantification is a very beneficial aspect of patient care. During a current state-of-the-art workflow, a user such as the ultrasound clinician first obtains ultrasound images, then hits the freeze button or its equivalent, at which point a quantification option such as a button is shown to the user. The user then selects the option, such as clicking the button, to initiate the quantification process. When complete, the result of the quantification - which can be any quantification of one or more of the obtained ultrasound images - is provided to the user via a user display of the ultrasound system.

As just one non-limiting example, for echocardiography, the sonographer may obtain 2D or 3D transthoracic apical 4-chamber (A4C) images of the heart, adhering to American Society of Echocardiography (ASE) guidelines (such as non-foreshortened image, sufficient endocardial border contrast visualized, vertical septum and adequate coverage of the image by the left ventricle (LV)). After obtaining the images, the sonographer then hits the freeze button, at which point the quantification button is shown to the user. The user would then click that button to initiate the quantification process. When complete, the result of the quantification - which can be demarcation of the LV in the 2D image, and can extrapolate that measurement to the third dimension, thereby measuring end diastolic volume (EDV), end systolic volume (ESV), stroke volume (SV), ejection fraction (EF), global longitudinal strain (GLS) and, if heart rate (HR) is available, then cardiac output (CO) as well - is provided to the user via a user display of the ultrasound system

However, this workflow relies on the sonographer to first acquire a high-quality loop and then initiate the auto-quantification module after the images are acquired. Prior art analysis solutions also either operate on-cart in a review mode or are available using off-cart analysis software. These prior art approaches are not particularly feasible in a critical care setting, however, since the time available to acquire images is minimal and patients are also difficult to image.

The document US2019130554A1 discloses systems and methods for calculating a quality of a sequence of images, determining whether the quality of the sequence of images exceeds a threshold quality, and performing an automatic measurement on the sequence of images based on determining that the quality of the sequence of images exceeds the threshold quality.

### Summary of the Disclosure

Accordingly, there is a continued need for methods and systems for ultrasound systems capable of quantifying patients in a fast-paced critical care setting. Embedding auto-quantification into a live-imaging workflow to prevent overhead for the sonographer and allow them to focus on the acquisition would facilitate these goals. Accordingly, various embodiments and implementations herein are directed to an ultrasound quantification method and system configured for automated ultrasound quantification. The ultrasound quantification system receives, from an ultrasound transducer probe, a plurality of ultrasound images of a subject's heart as a user is performing an ultrasound of the subject's heart. The system automatically provides, while the user is still performing the ultrasound of the subject's heart, some or all of the received plurality of ultrasound images of the subject's heart to a quantification algorithm. The quantification algorithm automatically performs a quantification analysis of the provided plurality of ultrasound images once a predetermined number of ultrasound images are provided to the quantification algorithm, without requiring a user activation or input to the quantification algorithm. The system them automatically notifies the user via a user interface of the ultrasound quantification system, while the user is still performing the ultrasound of the subject's heart, that a quantification of the provided plurality of ultrasound images is available for review. The system receives, from the user via the user interface, input directing the system to provide the quantification analysis to the user. The system then uses a user interface to provide the quantification analysis to a user.

Generally, in one aspect, a method for automated ultrasound quantification using a ultrasound quantification system comprising an ultrasound transducer probe is provided. The method includes: (i) receiving, from the ultrasound transducer probe, a plurality of ultrasound images of a subject as a user is performing an ultrasound of the subject; (ii) automatically providing, while the user is still performing the ultrasound of the subject, some or all of the received plurality of ultrasound images of the subject to a quantification algorithm; (iii) automatically performing, by the quantification algorithm, a quantification analysis of the provided plurality of ultrasound images once a predetermined number of ultrasound images are provided to the quantification algorithm, wherein the quantification analysis is performed without requiring a user activation or input to the quantification algorithm; (iv) automatically notifying the user via a user interface of the ultrasound quantification system, while the user is still performing the ultrasound of the subject, that a quantification of the provided plurality of ultrasound images is available for review; (v) receiving, from the user via the user interface, input directing the system to provide the quantification analysis to the user; and (vi) displaying, to the user via the user interface, the quantification analysis.

According to an embodiment, the ultrasound images are two-dimensional apical 4-chamber images. According to an embodiment, the plurality of ultrasound images are a video.

According to an embodiment, the method further includes receiving, from the user via the user interface, input modifying the quantification analysis. According to an embodiment, the input is a deletion of the quantification analysis. According to an embodiment, the input is an edit of the quantification analysis, and the method further includes modifying the quantification analysis based on the edit.

According to an embodiment, the method further includes receiving, from the user via the user interface, input to return the user to performing the ultrasound of the subject.

According to an embodiment, the method further includes analyzing the quantification algorithm's quantification of the provided plurality of ultrasound images to determine when the quantification satisfies a predetermined quality threshold, wherein the user is only notified that the quantification of the provided plurality of ultrasound images is available for review when the quantification satisfies the predetermined quality threshold.

According to an embodiment, the method further include storing the provided plurality of ultrasound images and/or the quantification analysis in a memory of the ultrasound quantification system.

According to an embodiment, the quantification analysis is displayed to the user via the user interface as one or more labeled images.

According to an embodiment, automatically notifying the user via a user interface that a quantification of the provided plurality of ultrasound images is available for review further comprises displaying a number of the subject's heart beats that have been quantified by the quantification analysis.

According to an embodiment, automatically notifying the user via a user interface that a quantification of the provided plurality of ultrasound images is available for review further comprises displaying an indicator of confidence in or quality of the quantification analysis.

According to another aspect is an ultrasound quantification system for automated ultrasound quantification. The system includes: an ultrasound transducer probe configured to obtain a plurality of ultrasound images of a subject; an ultrasound quantification algorithm configured to automatically perform a quantification analysis of a provided plurality of ultrasound images once a predetermined number of ultrasound images are provided to the quantification algorithm, wherein the quantification analysis is provided without requiring a user activation or input to the quantification algorithm; a user interface; and a processor configured to: (i) prompt the ultrasound quantification algorithm to perform a quantification analysis of a provided plurality of ultrasound images; (ii) prompt the user interface to automatically notify the user that a quantification of the provided plurality of ultrasound images is available for review; (iii) receive, via the user interface, input directing the system to provide the quantification analysis to the user; and (iv) prompting the user interface, in response to the received input, to display the quantification analysis. According to an embodiment, the processor is further configured to receive, via the user interface, input modifying the quantification analysis.

According to an embodiment, the processor is further configured to analyze the quantification algorithm's quantification of the provided plurality of ultrasound images to determine when the quantification satisfies a predetermined quality threshold, wherein the user is only notified that the quantification of the provided plurality of ultrasound images is available for review when the quantification satisfies the predetermined quality threshold.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### Brief Description of the Drawings

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
FIG. 1 is a flowchart of a method for automated ultrasound quantification, in accordance with an embodiment.
FIG. 2 is a schematic representation of an ultrasound quantification system, in accordance with an embodiment.
FIG. 3 is a flowchart of a method for automated ultrasound quantification, in accordance with an embodiment.
FIG. 4 is a flowchart of a method for automated ultrasound quantification, in accordance with an embodiment.
FIG. 5 is a flowchart of a method for automated ultrasound quantification, in accordance with an embodiment.

### Detailed Description of Embodiments

The present disclosure describes various embodiments of a system and method configured to perform ultrasound quantification. More generally, Applicant has recognized and appreciated that it would be beneficial to provide improved methods and systems for automated quantification of ultrasound images. Accordingly, an ultrasound quantification configured for automated ultrasound quantification receives, from an ultrasound transducer probe, a plurality of ultrasound images of a subject as a user is performing an ultrasound of the subject's heart. The system automatically provides, while the user is still performing the ultrasound of the subject, some or all of the received plurality of ultrasound images of the subject's heart to a quantification algorithm. The quantification algorithm atomically performs a quantification analysis of the provided plurality of ultrasound images once a predetermined number of ultrasound images are provided to the quantification algorithm, without requiring a user activation or input to the quantification algorithm. The system them automatically notifies the user via a user interface of the ultrasound quantification system, while the user is still performing the ultrasound of the subject, that a quantification of the provided plurality of ultrasound images is available for review. The system receives, from the user via the user interface, input directing the system to provide the quantification analysis to the user. The system then uses a user interface to provide the quantification analysis to a user.

According to an embodiment, the systems and methods described or otherwise envisioned herein can, in some non-limiting embodiments, be implemented as an element for a commercial product for ultrasound imaging or analysis, such as Philips^{®} Lumify^{®}, or as an element for a commercial product for cardiovascular analysis such as Philips IntelliSpace^{®} Cardiovascular (ISCV) (available from Koninklijke Philips NV, the Netherlands), or as an element for a commercial product for patient analysis or monitoring, such as the Philips Patient Flow Capacity Suite (PFCS), or any suitable system.

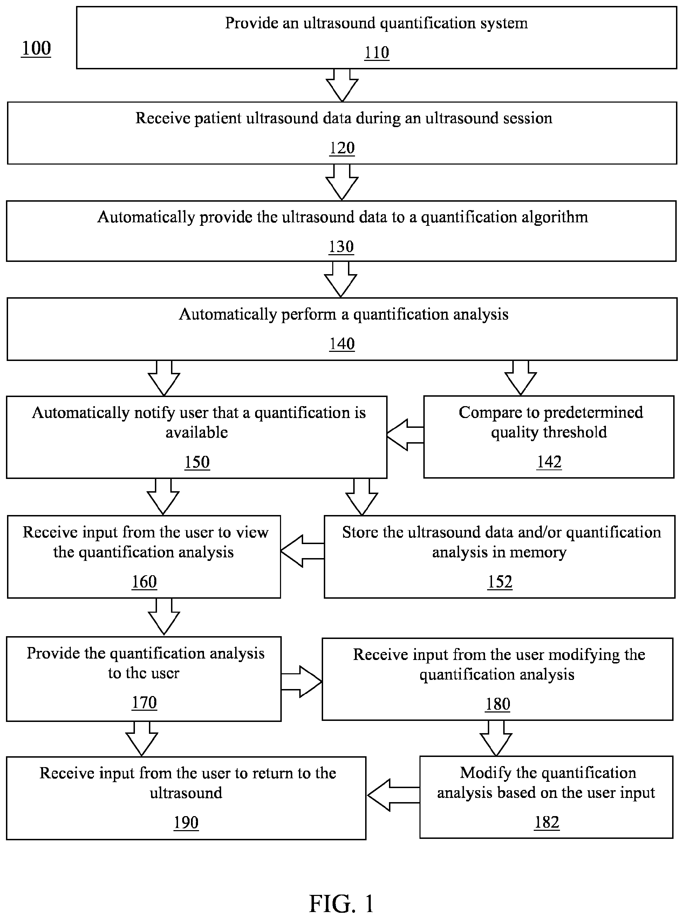

Referring to FIG. 1, in one embodiment, is a flowchart of a method 100 for automated ultrasound quantification using an ultrasound quantification system. The methods described in connection with the figures are provided as examples only, and shall be understood to not limit the scope of the disclosure. The ultrasound quantification system can be any of the systems described or otherwise envisioned herein. The ultrasound quantification system can be a single system or multiple different systems.

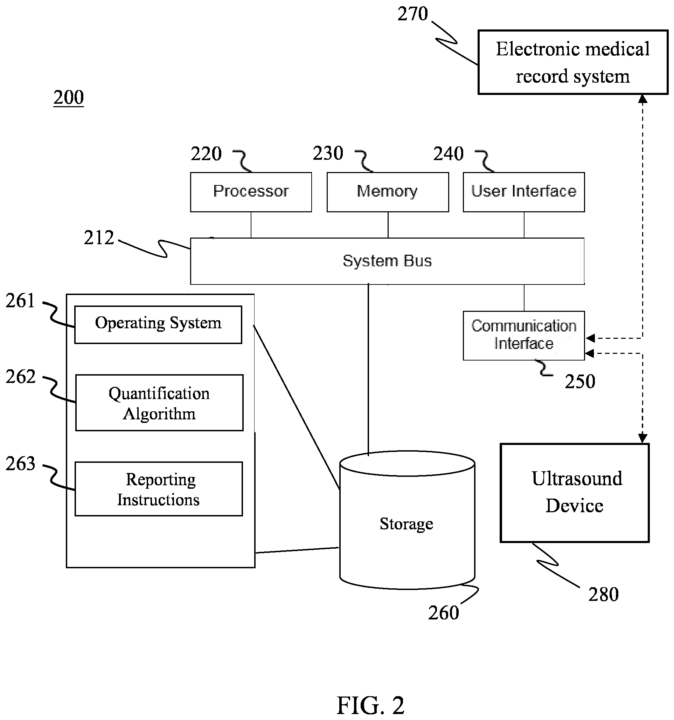

At step 110 of the method, an ultrasound quantification system 200 is provided. Referring to an embodiment of an ultrasound quantification system 200 as depicted in FIG. 2, for example, the system comprises one or more of a processor 220, memory 230, user interface 240, communications interface 250, and storage 260, interconnected via one or more system buses 212. It will be understood that FIG. 2 constitutes, in some respects, an abstraction and that the actual organization of the components of the system 200 may be different and more complex than illustrated. Additionally, ultrasound quantification system 200 can be any of the systems described or otherwise envisioned herein. Other elements and components of ultrasound quantification system 200 are disclosed and/or envisioned elsewhere herein.

At step 120 of the method, the ultrasound quantification system receives a plurality of ultrasound images of a subject's heart as a user is performing an ultrasound of the subject's heart. The user may be any user capable of or authorized to perform an ultrasound, such as a sonographer, nurse, physician, emergency medical technician, paramedic, or any other individual. The ultrasound image data may be obtained using any ultrasound device or system, which may be any device or system suitable to obtain or otherwise receive ultrasound image data of the patient. The ultrasound image data may be obtained as 2D or 3D data. The ultrasound image data may be obtained as video data. One or more parameters of the ultrasound device can be set, adjusted, preprogrammed, or otherwise determined by a healthcare professional. The ultrasound device or system may be remote to, local to, or a component of, the ultrasound quantification system. The ultrasound device or system comprises an ultrasound transducer probe configured to obtain the ultrasound images.

According to an embodiment, the ultrasound device or system is a stationary device, a portable device, or a handheld device. As just one non-limiting example, the ultrasound device or system is a smartphone connectable, app-based portable hand-held ultrasound device such as the Lumify Ultrasound System by Philips. Many other ultrasound devices and systems are possible.

According to an embodiment, the plurality of ultrasound images are obtained of any portion of the subject. For example, the ultrasound image data may be obtained of the subject's heart or any other portion of the subject. For example, the ultrasound image data may be two-dimensional apical 4-chamber images.

According to an embodiment, the ultrasound quantification system may comprise patient data about the subject for which an ultrasound exam will be performed. The patient data can be any information about the patient that the ultrasound quantification system can or may utilize for analysis as described or otherwise envisioned herein. According to an embodiment, the patient data comprises one or more of demographic information about the patient, medical history of the patient, a diagnosis for the patient, and a reason for the ultrasound to be performed. For example, demographic information may comprise information about the patient such as name, age, body mass index (BMI), and any other demographic information. The medical history of the patient may be any historical admittance or discharge information, historical treatment information, historical diagnosis information, historical exam or imaging information, and/or any other information. The diagnosis for the patient may be any information about a medical diagnosis for the patient, historical and/or current. The reason for the ultrasound to be performed may be any purpose, reason, need, or other impetus for the exam.

The patient data is received from one or a plurality of different sources. According to an embodiment, the patient data is received from, retrieved from, or otherwise obtained from an electronic medical record (EMR) database or system. The EMR database or system may be local or remote. The EMR database or system may be a component of the ultrasound quantification system, or may be in local and/or remote communication with the ultrasound quantification system. The received patient data may be utilized immediately, or may be stored in local or remote storage for use in further steps of the method.

At step 130 of the method, some or all of the received plurality of ultrasound images of the subject are automatically provided to a quantification algorithm, while the user is still performing the ultrasound of the subject. Thus, some or all of the images are sent to the algorithm in the background, while the user is performing more of the ultrasound of the subject.

According to an embodiment, the ultrasound system comprises one or more quantification algorithms. Alternatively, an ultrasound system may be in communication with the ultrasound quantification system which comprises the ultrasound quantification algorithm. For example, an ultrasound device may send images to a local or remote device such as a smartphone or other computer, and the smartphone or other computer may comprise a quantification algorithm.

According to an embodiment, the quantification algorithm is configured to analyze the received ultrasound images to provide quantification information to a user. For example, a quantification algorithm may be programmed or taught or otherwise designed to segment or demarcate physiological structures in obtained images, and may then make quantitative measurements of size, volume, and other physiological parameters. For example, a quantification algorithm for ultrasound images of a heart may demarcate the left ventricle (LV) in one or more 2D images and extrapolate that measurement to the third dimension, thereby measuring end diastolic volume (EDV), end systolic volume (ESV), stroke volume (SV), ejection fraction (EF), global longitudinal strain (GLS) and, if heart rate (HR) is available, then cardiac output (CO) as well. Many other quantification algorithms are possible.

At step 140 of the method, the quantification algorithm of the ultrasound quantification system automatically performs a quantification analysis of the provided plurality of ultrasound images. The quantification analysis can be any quantification analysis performed by any quantification algorithm, including as described or otherwise envisioned herein. According to one embodiment, the quantification algorithm automatically performs the quantification analysis once a predetermined number of ultrasound images are provided to the quantification algorithm. For example, the quantification algorithm or ultrasound quantification system may count or track the number of images or frames obtained by the device, and may automatically trigger a quantification analysis once a predetermined number of images or frames are obtained. According to another embodiment, the quantification algorithm automatically performs the quantification analysis once one or more ultrasound images of a predetermined quality are provided to the quantification algorithm. For example, the quantification algorithm or ultrasound quantification system may analyze the obtained plurality of ultrasound images for quality, where quality may be programmed, learned, or otherwise determined. The quality may depend on the presence of one or more physiological structures in the images, among many other parameters.

Notably, the quantification algorithm of the ultrasound quantification system automatically performs the quantification analysis without requiring any user activation or input to either the quantification algorithm or the ultrasound quantification system. In this way, the quantification algorithm performs the analysis in the background, while the user is still performing some or all of the remainder of the ultrasound.

Accordingly, the quantification algorithm of the ultrasound quantification system generates a quantification analysis that is prepared for review by the user, or by another individual, comprising any information that is or can be generated by quantification algorithm about the plurality of obtained ultrasound images.

At optional step 142 of the method, the quantification algorithm and/or the ultrasound quantification system analyzes the generated quantification analysis to determine when that quantification analysis satisfies a predetermined quality threshold. For example, the system may wait to proceed to a next step in the method or process until a certain number of images are analyzed by the quantification algorithm, until a predetermined number of physiological parameters are determined or measured by the quantification algorithm, until one or more specific physiological parameters are determined or measured by the quantification algorithm, or until another predetermined trigger is satisfied or occurs.

At step 150 of the method, the ultrasound quantification system automatically notifies the user that a quantification of the provided plurality of ultrasound images is available for review. This notification is provided to the user while the use is still performing at least a portion of the ultrasound of the patient. Thus, the quantification algorithm of the system has automatically performed the quantification analysis and it is ready for the user to review. If the system implements a quality check on the quantification analysis, the notification is provided to the user if the quantification analysis passes the quality check.

Importantly, the ultrasound quantification system provides the notification that the quantification analysis is available without requiring any input from the user. For example, the user does not need to pause the ultrasound exam and check another screen, page, window, or other area to determine whether an ultrasound quantification is available for review. This saves the user from wasting valuable ultrasound exam time to check whether an ultrasound quantification is available for review, which could happen multiple times during a single exam.

According to an embodiment, the automatic notification is provided via a user interface of the ultrasound quantification system, such as via a screen including the ultrasound exam window, among other screens or other notification interfaces. Alternatively, the automatic notification is provided via another computer or system, such as a smartphone, watch, wearable device, or other user interface. The automatic notification can be any mechanism for providing information to a user. For example, the automatic notification can be a pop-up, a new or changed icon or button, a highlighted icon or button, a haptic notification, a noise, a message, or any other notification.

According to an embodiment, the automatic notification further comprises displaying an indicator of confidence in or quality of the quantification analysis. According to an embodiment, the automatic notification further comprises displaying a number of the subject's heart beats that have been quantified by the quantification analysis. This could be, for example, a number such as an icon, among other display mechanisms.

At step 152 of the method, the ultrasound quantification system stores the provided plurality of ultrasound images and/or the generated quantification analysis in a memory of the ultrasound quantification system. The system may also store information about the patient, about the user, or about any other aspect of the ultrasound exam and analysis. The memory may be a component of the ultrasound quantification system, or may be in local and/or remote communication with the ultrasound quantification system. The stored data may be utilized immediately, or may be stored in local or remote storage for use in further steps of the method.

At step 160 of the method, the ultrasound quantification system receives input from the user directing the system to provide the quantification analysis to the user. In other words, the system notifies the user that a quantification analysis is available to review, and waits for input from the user to show that quantification analysis to the user. Thus, the user can decide whether or when it is an appropriate time to pause or interrupt the ultrasound exam to review the ultrasound quantification analysis. The input can be received via any user interface, whether a component of the ultrasound quantification system or a user interface in communication with the ultrasound quantification system. For example, if the notification is provided via a screen such as the ultrasound exam window, the user can click or touch or voice-select the notification or another button or interactive element in order to direct the system to provide the quantification analysis. As another example, if the automatic notification is provided via another computer or system, such as a smartphone, watch, wearable device, or other user interface, the user can click or touch or voice-select that notification in order to direct the system to provide the quantification analysis.

At step 170 of the method, the ultrasound quantification system provides the quantification analysis to the user via a user interface. The quantification analysis can be provided to the user via any known mechanism for providing quantification analyses. For example, the quantification analysis may be provided on the ultrasound exam window, in another window or on another screen, as a visual display such as a projector or a wearable device, and via any other mechanism. The displayed quantification analysis can comprise any of the elements of the quantification analysis, and may optionally include other information such as patient demographics or medical information, and/or any other information. As an example, the quantification analysis can be displayed to the user via the user interface as one or more labeled images.

At step 180 of the method, the ultrasound quantification system provides receives input from the user modifying the quantification analysis. The modification input can be received via a user interface of the system, such as a window, screen, or other display, whether the user interface is a whether a component of the ultrasound quantification system or a user interface in communication with the ultrasound quantification system. The input can be provided by any mechanism for using a user interface to provide feedback. For example, the user can click or touch or voice-select a button or interactive element in order to provide modification input for the quantification analysis. As another example, if the user interface is provided via another computer or system, such as a smartphone, watch, wearable device, or other user interface, the user can click or touch or voice-select a button or interactive element of that other computer or system in order to provide modification input.

According to an embodiment, the modification input can be any command or direction from the user to modify the quantification analysis. For example, the user may alter a quantification decision by the quantification analysis, correct or modify a parameter or demarcation by the quantification analysis, and/or make any other modifying input. According to an embodiment, the modification input comprises a command or direction from the user to save or delete or refresh or update the quantification analysis.

As another example, the modification input comprises a command or direction from the user to edit the quantification analysis. Accordingly, at step 182 of the method, the ultrasound quantification system implements the command or direction provided by the user. For example, the modification input may be a command or direction to alter a quantification decision by the quantification analysis, correct or modify a parameter or demarcation by the quantification analysis, or a command or direction from the user to save or delete or refresh or update the quantification analysis. Upon receive the modification input, the ultrasound quantification system can implement the modification input accordingly.

At step 190 of the method, the ultrasound quantification system receives input from the user directing the system to return to a status such that the user can continue to perform the ultrasound of the patient. This can be, for example, as simple as minimizing or removing a window or display showing the quantification analysis. As another example, returning the system to a status such that the user can continue to perform the ultrasound of the patient can be more complicated, such as closing one system and re-opening or otherwise returning to another system. The input can be received from the user via a user interface of the system, such as a window, screen, or other display, whether the user interface is a whether a component of the ultrasound quantification system or a user interface in communication with the ultrasound quantification system. The input can be provided by any mechanism for using a user interface to provide feedback. For example, the user can click or touch or voice-select a button or interactive element. As another example, if the user interface is provided via another computer or system, such as a smartphone, watch, wearable device, or other user interface, the user can click or touch or voice-select a button or interactive element of that other computer or system.

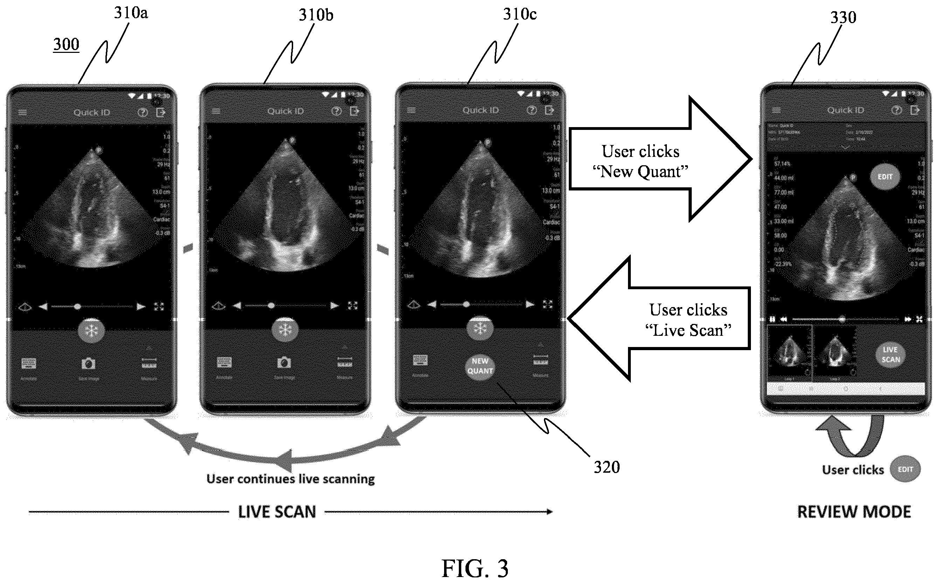

Referring to FIG. 3, in one embodiment, is a flowchart of a method 300 for automated ultrasound quantification using an ultrasound quantification system. In this embodiment, the user views ultrasound imagery and an ultrasound quantification, and interacts with the ultrasound quantification system, via a handheld device such as a smartphone, tablet, or other device. However, the display and user interface of the ultrasound quantification system can be any possible display or user interface. Further, in this example the user is obtaining ultrasound imagery of the patient's heart, although the ultrasound quantification system can be utilized for any ultrasound analysis.

According to an embodiment, the user begins a live scan and attempts to get a transthoracic A4C view of the patient's heart. The different images (L-R 310a, 310b, 310c) in the live scan mode represent different time points during the live scan. When certain conditions are satisfied as described or otherwise envisioned herein, a quantification(s) becomes available, and an indicator/button 320 automatically shows up on the live scan user interface, without any user input. When the user clicks that button, the system transitions to a Review Mode 330 and shows all available quantifications as thumbnails, with the first one auto-selected for full display. The user can either edit the auto-contours (and the quantifications will be auto-updated as a result), or if they are satisfied with the results, can return to live scan mode at any time (such as by clicking the "Live Scan" button in window 330. There is also the option to delete the quantification(s). As is appreciable, the user can enter the Review mode at their own volition to review the quantifications. This can also happen at the end of the scan. Note that the user does not need to take any explicit actions to perform the quantifications - it is a seamless process happening automatically during live scanning.

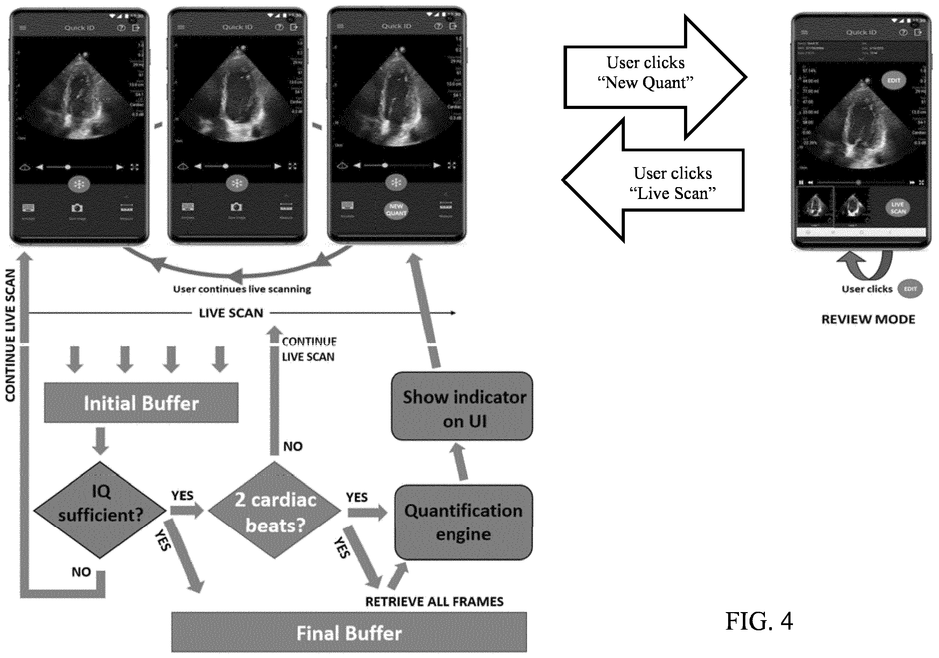

Referring to FIG. 4, in one embodiment, as the user performs a live scan, the acquired frames are continuously fed to a "quantification pipeline" which comprises an initial image quality (IQ) check, followed by triggering of the quantification algorithm if sufficient successive frames are available such as, for example, at least 2 cardiac beats, although more or less are possible. According to an embodiment, the at least 2 cardiac beat segments sequentially fed into the quantification engine such that there can be overlap (i.e. beat 1+2, beat 2+3, beat 3+4, or: 1+2+3+4 , 3+4+5+6, 5+6+7+8, etc.).

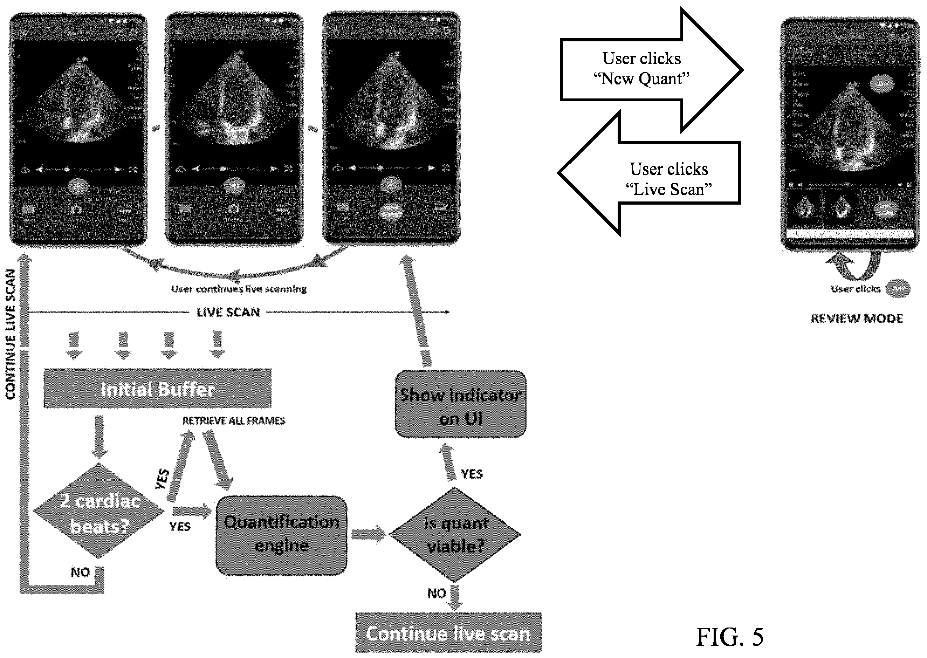

Referring to FIG. 5, in one embodiment, as the user performs a live scan, the acquired frames are continuously fed to a "quantification pipeline", which when sufficient successive frames are available (such as, for example, at least 2 cardiac beats, although more or less are possible) triggers the quantification algorithm. The results of the quantification algorithm are then checked for viability, and if considered viable, are kept, or else they are discarded.

According to an embodiment, for each successful quantification, at least two items are saved to the system memory: (i) all of the image frames that were used for the quantification (called "image loop"); and (ii) all the numerics that were derived from the quantification algorithm (and, for example, tagged to the corresponding image loop).

According to an embodiment, if at least one quantification is available, then the user is notified via a button (or other indicator) on the live scan screen of the system. The user can click that button to transition them to a "review" mode, in which they can review all quantifications (and the underlying image loops) acquired so far, and either accept as is, edit or reject each one.

According to an embodiment, the notification button can show a visible integer number indicating how many beats have been viably quantified so far. The beat counter can reset to zero every time the notification button is pressed, and can be continuously updated until pressed.

According to an embodiment, some or all of the notification button can include a color that is indicative of most recent image quality. For example, the color could be directly tied to the output of the initial image quality check. As another example, the color could be tied to the quality of latest output from the quantification engine (i.e., orange if confidence is low, green if confidence is high, etc.).

According to an embodiment, an indication can be provided to the user that automatic acquisition with sufficient image quality has commenced while also indicating progress of acquiring the required or desired number of beats, e.g. via a circle that fills in a clockwise manner to indicate how many percent of the required or desired number of beats have been acquired. This will prompt the user to hold the probe still and improve acquisition success rate.

Referring to FIG. 2 is a schematic representation of an ultrasound quantification system 200. System 200 may be any of the systems described or otherwise envisioned herein, and may comprise any of the components described or otherwise envisioned herein. It will be understood that FIG. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, the ultrasound quantification system comprises or is in communication with an electronic medical record system 270 which is an electronic medical records database from which the information about the patient, including clinical information and ultrasound data, may be obtained or received. The electronic medical records database may be a local or remote database and is in direct and/or indirect communication with the ultrasound quantification system 200. Thus, according to an embodiment, the ultrasound quantification system comprises an electronic medical record database or system 270.

According to an embodiment, the system comprises one or more ultrasound devices 280 capable of acquiring the required ultrasound images or analysis. According to another embodiment, ultrasound quantification system 200 is in wired and/or wireless communication with a local or remote ultrasound device 280 capable of acquiring the required ultrasound images or analysis.

According to an embodiment, storage 260 of system 200 may store one or more algorithms, modules, and/or instructions to carry out one or more functions or steps of the methods described or otherwise envisioned herein. For example, the system may comprise, among other instructions or data, a quantification algorithm 262 and/or reporting instructions 263.

According to an embodiment, the quantification algorithm 262 is any program or algorithm is configured to analyze the received ultrasound images to provide quantification information to a user. For example, a quantification algorithm may be programmed or taught or otherwise designed to segment or demarcate physiological structures in obtained images, and may then make quantitative measurements of size, volume, and other physiological parameters. For example, a quantification algorithm for ultrasound images of a heart may demarcate the left ventricle (LV) in one or more 2D images and extrapolate that measurement to the third dimension, thereby measuring end diastolic volume (EDV), end systolic volume (ESV), stroke volume (SV), ejection fraction (EF), global longitudinal strain (GLS) and, if heart rate (HR) is available, then cardiac output (CO) as well. Many other quantification algorithms are possible.

According to an embodiment, reporting instructions 263 direct the system to generate and provide to a user via a user interface information comprising the notification that the quantification analysis is available for review. The reporting instructions 263 may also direct the system to provide the quantification analysis to the user for review. Information may be provided to a user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

Accordingly, within the context of the disclosure herein, aspects of the embodiments may take the form of a computer program product embodied in one or more non-transitory computer readable media having computer readable program code embodied thereon. Thus, according to one embodiment is a non-transitory computer-readable storage medium comprising computer program code instructions which, when executed by a processor, enables the processor to carry out a method including: (i) receiving a plurality of ultrasound images of a subject's heart as a user is performing an ultrasound of the subject's heart; (ii) automatically providing, while the user is still performing the ultrasound of the subject's heart, some or all of the received plurality of ultrasound images of the subject's heart to a quantification algorithm; (iii) automatically performing, by the quantification algorithm, a quantification analysis of the provided plurality of ultrasound images once a predetermined number of ultrasound images are provided to the quantification algorithm, wherein the quantification analysis is provided without requiring a user activation or input to the quantification algorithm; (iv) automatically notifying the user via a user interface of the ultrasound quantification system, while the user is still performing the ultrasound of the subject's heart, that a quantification of the provided plurality of ultrasound images is available for review; (v) receiving, from the user via the user interface, input directing the system to provide the quantification analysis to the user; and (vi) displaying, to the user via the user interface, the quantification analysis. The program code may perform entirely on a user's computer, partly on a user's computer, as a stand-alone software package, partly on a user's computer and partly on a remote computer, or entirely on a remote computer or server.

According to an embodiment, the ultrasound quantification system is configured to process many thousands or millions of datapoints to process and analyze the plurality of ultrasound images using the quantification algorithm, as well as to provide input to the user, receive input from the user, and potentially modify the quantification. Indeed, generating a quantification analysis is a process that has been relegated to algorithms because the human mind is incapable or performing the analysis in the timeframe required or with the accuracy required. This requires millions or billions of calculations to generate a quantification analysis. By providing an improve ultrasound quantification system that automatically generates a quantification analysis and automatically notifies a user that it is ready for review, the system has an enormous positive effect on patient analysis and care by saving user time and keeping the user free from distraction, compared to prior art systems.

The present invention may be a system, a method, and/or a computer program product. The computer program product may include a non-transitory computer readable storage medium (or media) having computer readable program instructions thereon for causing a system or processor to carry out aspects of the present invention. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any combination of the foregoing, among other possibilities. Computer readable program instructions described herein can be downloaded to respective computing/processing devices from a computer readable storage medium or to an external computer or external storage device via a network, for example, the internet, a local area network, and/or a wireless network. Aspects of the present invention are described herein with reference to flowchart illustrations and/or block diagrams of methods, apparatus, systems, and computer program products according to embodiments of the invention. It will be understood that each block of the flowchart illustrations and/or block diagrams, and combinations of blocks in the flowchart illustrations and/or block diagrams, can be implemented by computer readable program instructions.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for automated ultrasound quantification using an ultrasound quantification system (200) comprising an ultrasound transducer probe (270), the method comprising:
receiving (120), from the ultrasound transducer probe, a plurality of ultrasound images of a subject as a user is performing an ultrasound of the subject;
automatically providing (130), while the user is still performing the ultrasound of the subject, some or all of the received plurality of ultrasound images of the subject to a quantification algorithm;
automatically performing (140), by the quantification algorithm, a quantification analysis of the provided plurality of ultrasound images once a predetermined number of ultrasound images are provided to the quantification algorithm, wherein the quantification analysis is performed without requiring a user activation or input to the quantification algorithm;
automatically notifying (150) the user via a user interface (240) of the ultrasound quantification system, while the user is still performing the ultrasound of the subject, that a quantification of the provided plurality of ultrasound images is available for review;
receiving (160), from the user via the user interface, input directing the system to provide the quantification analysis to the user; and
displaying (170), to the user via the user interface, the quantification analysis.

2. The method of claim 1, wherein the ultrasound images are two-dimensional apical 4-chamber images.

3. The method of claim 1, wherein the plurality of ultrasound images are a video.

4. The method of claim 1, further comprising the step of receiving (180), from the user via the user interface, input modifying the quantification analysis.

5. The method of claim 4, wherein the input is a deletion of the quantification analysis.

6. The method of claim 4, wherein the input is an edit of the quantification analysis, and further comprising the step of (182) modifying the quantification analysis based on the edit.

7. The method of claim 1, further comprising the step of receiving (190), from the user via the user interface, input to return the user to performing the ultrasound of the subject.

8. The method of claim 1, further comprising the step of:
analyzing (142) the quantification algorithm's quantification of the provided plurality of ultrasound images to determine when the quantification satisfies a predetermined quality threshold, wherein the user is only notified that the quantification of the provided plurality of ultrasound images is available for review when the quantification satisfies the predetermined quality threshold.

9. The method of claim 1, further comprising the step of storing (152) the provided plurality of ultrasound images and/or the quantification analysis in a memory of the ultrasound quantification system.

10. The method of claim 1, wherein the quantification analysis is displayed to the user via the user interface as one or more labeled images.

11. The method of claim 1, wherein automatically notifying the user via a user interface that a quantification of the provided plurality of ultrasound images is available for review further comprises displaying a number of the subject's heart beats that have been quantified by the quantification analysis.

12. The method of claim 1, wherein automatically notifying the user via a user interface that a quantification of the provided plurality of ultrasound images is available for review further comprises displaying an indicator of confidence in or quality of the quantification analysis.

13. An ultrasound quantification system (200) for automated ultrasound quantification, comprising:
an ultrasound transducer probe (270) configured to obtain a plurality of ultrasound images of a subject;
an ultrasound quantification algorithm (262) configured to automatically perform a quantification analysis of a provided plurality of ultrasound images once a predetermined number of ultrasound images are provided to the quantification algorithm, wherein the quantification analysis is provided without requiring a user activation or input to the quantification algorithm;
a user interface (240);
a processor (220) configured to: (i) prompt the ultrasound quantification algorithm to perform a quantification analysis of a provided plurality of ultrasound images; (ii) prompt the user interface to automatically notify the user that a quantification of the provided plurality of ultrasound images is available for review; (iii) receive, via the user interface, input directing the system to provide the quantification analysis to the user; and (iv) prompting the user interface, in response to the received input, to display the quantification analysis.

14. The system of claim 13, wherein the processor is further configured to receive, via the user interface, input modifying the quantification analysis.

15. The system of claim 13, wherein the processor is further configured to analyze the quantification algorithm's quantification of the provided plurality of ultrasound images to determine when the quantification satisfies a predetermined quality threshold, wherein the user is only notified that the quantification of the provided plurality of ultrasound images is available for review when the quantification satisfies the predetermined quality threshold.

## Patentansprüche

1. Verfahren (100) zur automatisierten Ultraschallquantifizierung unter Verwendung eines Ultraschallquantifizierungssystems (200), das eine Ultraschallwandlersonde (270) umfasst, wobei das Verfahren Folgendes umfasst:
Empfangen (120) von der Ultraschallwandlersonde einer Vielzahl von Ultraschallbildern eines Subjekts, während ein Benutzer eine Ultraschalluntersuchung des Subjekts durchführt;
automatisches Bereitstellen (130), während der Benutzer noch die Ultraschalluntersuchung des Subjekts durchführt, einiger oder aller der empfangenen Vielzahl von Ultraschallbildern des Subjekts einem Quantifizierungsalgorithmus;
automatisches Durchführen (140), durch den Quantifizierungsalgorithmus, einer Quantifizierungsanalyse der bereitgestellten Vielzahl von Ultraschallbildern, sobald dem Quantifizierungsalgorithmus eine vorbestimmte Anzahl von Ultraschallbildern bereitgestellt wird, wobei die Quantifizierungsanalyse ohne eine Aktivierung oder Eingabe des Quantifizierungsalgorithmus durch den Benutzer zu erfordern, durchgeführt wird;
automatisches Benachrichtigen (150) des Benutzers über eine Benutzeroberfläche (240) des Ultraschallquantifizierungssystems, während der Benutzer noch die Ultraschalluntersuchung des Subjekts durchführt, dass eine Quantifizierung der bereitgestellten Vielzahl von Ultraschallbildern zur Überprüfung verfügbar ist;
Empfangen (160) einer Eingabe des Benutzers über die Benutzeroberfläche, die das System anweist, dem Benutzer die Quantifizierungsanalyse bereitzustellen; und
Anzeigen (170) der Quantifizierungsanalyse dem Benutzer über die Benutzeroberfläche.

2. Verfahren nach Anspruch 1, wobei die Ultraschallbilder zweidimensionale apikale 4-Kammer-Bilder sind.

3. Verfahren nach Anspruch 1, wobei die Vielzahl von Ultraschallbildern ein Video ist.

4. Verfahren nach Anspruch 1, weiter umfassend den Schritt zum Empfangen (180) einer Eingabe zum Modifizieren der Quantifizierungsanalyse von dem Benutzer über die Benutzeroberfläche.

5. Verfahren nach Anspruch 4, wobei die Eingabe eine Löschung der Quantifizierungsanalyse ist.

6. Verfahren nach Anspruch 4, wobei die Eingabe eine Bearbeitung der Quantifizierungsanalyse ist, und weiter umfassend den Schritt (182) zum Modifizieren der Quantifizierungsanalyse auf der Grundlage der Bearbeitung.

7. Verfahren nach Anspruch 1, weiter umfassend den Schritt zum Empfangen (190) einer Eingabe von dem Benutzer über die Benutzeroberfläche, um den Benutzer zum Durchführen der Ultraschalluntersuchung des Subjekts zurückzuführen.

8. Verfahren nach Anspruch 1, weiter umfassend den Schritt zum
Analysieren (142) der Quantifizierung der bereitgestellten Vielzahl von Ultraschallbildern durch den Quantifizierungsalgorithmus, um zu bestimmen, wann die Quantifizierung einen vorbestimmten Qualitätsschwellenwert erfüllt, wobei der Benutzer nur dann benachrichtigt wird, dass die Quantifizierung der bereitgestellten Vielzahl von Ultraschallbildern zur Überprüfung verfügbar ist, wenn die Quantifizierung den vorbestimmten Qualitätsschwellenwert erfüllt.

9. Verfahren nach Anspruch 1, weiter umfassend den Schritt zum Speichern (152) der bereitgestellten Vielzahl von Ultraschallbildern und/oder der Quantifizierungsanalyse in einem Speicher des Ultraschallquantifizierungssystems.

10. Verfahren nach Anspruch 1, wobei die Quantifizierungsanalyse dem Benutzer über die Benutzeroberfläche als ein oder mehrere beschriftete Bilder angezeigt wird.

11. Verfahren nach Anspruch 1, wobei automatisches Benachrichtigen des Benutzers über eine Benutzeroberfläche, dass eine Quantifizierung der bereitgestellten Vielzahl von Ultraschallbildern zur Überprüfung verfügbar ist, weiter Anzeigen einer Anzahl der Herzschläge des Subjekts umfasst, die durch die Quantifizierungsanalyse quantifiziert wurden.

12. Verfahren nach Anspruch 1, wobei automatisches Benachrichtigen des Benutzers über eine Benutzeroberfläche, dass eine Quantifizierung der bereitgestellten Vielzahl von Ultraschallbildern zur Überprüfung verfügbar ist, weiter Anzeigen eines Indikators eines Vertrauens in die oder einer Qualität der Quantifizierungsanalyse umfasst.

13. Ultraschallquantifizierungssystem (200) zur automatisierten Ultraschallquantifizierung, umfassend:
eine Ultraschallwandlersonde (270), die so konfiguriert ist, dass sie eine Vielzahl von Ultraschallbildern eines Subjekts erhält;
einen Ultraschallquantifizierungsalgorithmus (262), der so konfiguriert ist, dass er automatisch eine Quantifizierungsanalyse einer bereitgestellten Vielzahl von Ultraschallbildern durchführt, sobald dem Quantifizierungsalgorithmus eine vorbestimmte Anzahl von Ultraschallbildern bereitgestellt wird, wobei die Quantifizierungsanalyse bereitgestellt wird, ohne Aktivierung oder Eingabe durch den Benutzer zu erfordern;
eine Benutzeroberfläche (240);
einen Prozessor (220), der so konfiguriert ist, dass er: (i) den Ultraschallquantifizierungsalgorithmus auffordert, eine Quantifizierungsanalyse einer bereitgestellten Vielzahl von Ultraschallbildern durchzuführen; (ii) die Benutzeroberfläche auffordert, den Benutzer automatisch zu benachrichtigen, dass eine Quantifizierung der bereitgestellten Vielzahl von Ultraschallbildern zur Überprüfung verfügbar ist; (iii) über die Benutzeroberfläche Eingaben empfängt, die das System anweisen, dem Benutzer die Quantifizierungsanalyse bereitzustellen; und (iv) die Benutzeroberfläche auffordert, als Reaktion auf die empfangenen Eingaben die Quantifizierungsanalyse anzuzeigen.

14. System nach Anspruch 13, wobei der Prozessor weiter so konfiguriert ist, dass er über die Benutzeroberfläche eine Eingabe empfängt, die die Quantifizierungsanalyse modifiziert.

15. System nach Anspruch 13, wobei der Prozessor weiter so konfiguriert ist, dass er die Quantifizierung der bereitgestellten Vielzahl von Ultraschallbildern durch den Quantifizierungsalgorithmus analysiert, um zu bestimmen, wann die Quantifizierung einen vorbestimmten Qualitätsschwellenwert erfüllt, wobei der Benutzer nur dann benachrichtigt wird, dass die Quantifizierung der bereitgestellten Vielzahl von Ultraschallbildern zur Überprüfung verfügbar ist, wenn die Quantifizierung den vorbestimmten Qualitätsschwellenwert erfüllt.

## Revendications

1. Procédé (100) de quantification par ultrasons automatisée utilisant un système de quantification par ultrasons (200) comprenant une sonde transducteur à ultrasons (270), le procédé comprenant :
la réception, (120), depuis la sonde tranducteur à ultrasons, d'une pluralité d'images échographiques d'un sujet pendant qu'un utilisateur effectue une échographie du sujet ;
la fourniture automatique (130), pendant que l'utilisateur effectue encore l'échographie du sujet, d'une partie ou de la totalité de la pluralité reçue d'images échographiques du sujet à un algorithme de quantification ;
la réalisation automatique (140), par l'intermédiaire de l'algorithme de quantification, d'une analyse de quantification de la pluralité fournie d'images échographiques une fois qu'un nombre prédéterminé d'images échographiques sont fournies à l'algorithme de quantification, dans lequel l'analyse de quantification est effectuée sans nécessiter d'activation ou d'entrée d'utilisateur dans l'algorithme de quantification ;
la notification automatique (150) de l'utilisateur via une interface utilisateur (240) du système de quantification par ultrasons, pendant que l'utilisateur effectue encore l'échographie du sujet, qu'une quantification de la pluralité fournie d'images échographiques est disponible pour examen ;
la réception (160), à parir de l'utilisateur via l'interface utilisateur, d'une entrée indiquant au système de fournir l'analyse de quantification à l'utilisateur ; et
l'affichage (170), à l'utilisateur via l'interface utilisateur, de l'analyse de quantification.

2. Procédé selon la revendication 1, dans lequel les images échographiques sont des images apicales bidimensionnelles à 4 chambres.

3. Procédé selon la revendication 1, dans lequel la pluralité d'images échographiques est une vidéo.

4. Procédé selon la revendication 1, comprenant en outre l'étape de réception (180), de la part de
l'utilisateur, via l'interface utilisateur, d'une entrée modifiant l'analyse de quantification.

5. Procédé selon la revendication 4, dans lequel l'entrée est une suppression de l'analyse de quantification.

6. Procédé selon la revendication 4, dans lequel l'entrée est une édition de l'analyse de quantification, et comprenant en outre l'étape (182) de modification de l'analyse de quantification sur la base de l'édition.

7. Procédé selon la revendication 1, comprenant en outre l'étape de réception (190), de la part de l'utilisateur via l'interface utilisateur, d'une entrée pour permettre à l'utilisateur de revenir à la réalisation de l'échographie du sujet.

8. Procédé selon la revendication 1, comprenant en outre l'étape de :
analyse (142) de la quantification par l'algorithme de quantification de la pluralité fournie d'images échographiques afin de déterminer quand la quantification satisfait un seuil de qualité prédéterminé, dans lequel l'utilisateur n'est informé que la quantification de la pluralité fournie d'images échographiques est disponible pour examen que lorsque la quantification satisfait le seuil de qualité prédéterminé.

9. Procédé selon la revendication 1, comprenant en outre l'étape de stockage (152) de la pluralité fournie d'images échographiques et/ou l'analyse de quantification dans une mémoire du système de quantification par ultrasons.

10. Procédé selon la revendication 1, dans lequel l'analyse de quantification est affichée à l'utilisateur via l'interface utilisateur sous forme d'une ou plusieurs images étiquetées.

11. Procédé selon la revendication 1, dans lequel la notification automatique à l'utilisateur, via une interface utilisateur, qu'une quantification de la pluralité fournie d'images échographiques est disponible pour examen, comprend en outre un affichage d'un certain nombre de battements cardiaques du sujet qui ont été quantifiés par l'analyse de quantification.

12. Procédé selon la revendication 1, dans lequel la notification automatique à l'utilisateur, via une interface utilisateur, qu'une quantification de la pluralité fournie d'images échographiques est disponible pour examen, comprend en outre un affichage d'un indicateur de confiance ou de qualité de l'analyse de quantification.

13. Système de quantification par ultrasons (200) pour une quantification par ultrasons automatisée, comprenant :
une sonde transducteur à ultrasons (270) configurée pour obtenir une pluralité d'images à ultrasons d'un sujet ;
un algorithme de quantification par ultrasons (262) configuré pour effectuer automatiquement une analyse de quantification d'une pluralité fournie d'images échographiques une fois qu'un nombre prédéterminé d'images échographiques est fourni à l'algorithme de quantification, dans lequel l'analyse de quantification est fournie sans nécessiter d'activation ni d'entrée d'utilisateur dans l'algorithme de quantification ;
une interface utilisateur (240) ;
un processeur (220) configuré pour : (i) demander à l'algorithme de quantification par ultrasons d'effectuer une analyse de quantification d'une pluralité fournie d'images échographiques ; (ii) demander à l'interface utilisateur de notifier automatiquement à l'utilisateur qu'une quantification de la pluralité fournie d'images échographiques est disponible pour examen ; (iii) recevoir, via l'interface utilisateur, une entrée ordonnant au système de fournir l'analyse de quantification à l'utilisateur ; et (iv) inviter l'interface utilisateur, en réponse à l'entrée reçue, d'afficher l'analyse de quantification.

14. Système selon la revendication 13, dans lequel le processeur est en outre configuré pour recevoir, via l'interface utilisateur, des entrées modifiant l'analyse de quantification.

15. Système selon la revendication 13, dans lequel le processeur est en outre configuré pour analyser la quantification par l'algorithme de quantification de la pluralité fournie d'images échographiques afin de déterminer quand la quantification satisfait un seuil de qualité prédéterminé, dans lequel l'utilisateur n'est notifié que la quantification de la pluralité fournie d'images échographiques est disponible pour examen que lorsque la quantification satisfait le seuil de qualité prédéterminé.
